# EUROPEAN PATENT APPLICATION

(11) **EP 4 785 989 A1**
(43) Date of publication of application: **05.08.2026**
(21) Application number: 25155691.6
(22) Date of filing: 04.02.2025
(51) Int. Cl.: A61N 1/05

(54) **FIXATION SLEEVE AND ASSEMBLY COMPRISING SUCH SLEEVE**

(71) Applicant: BIOTRONIK SE & Co. KG, 12359 Berlin (DE)
(72) Inventor: TRÖGER, Uwe, 12351 Berlin (DE)
(74) Representative: Biotronik Corporate Services SE

(57) **Abstract**

The invention relates to a fixation sleeve for an implantable lead with a lead body, wherein the fixation sleeve has a substantially tubular shape comprising an internal lumen extending therethrough and extending in a longitudinal direction. To achieve a secure and reproducible fixation of the fixation sleeve to the lead body even if the lead body slightly varies in diameter, the fixation sleeve comprises a tubular mesh configured such that an internal mesh lumen forms a section of the internal lumen of the fixation sleeve, wherein the internal mesh lumen is configured to receive the lead body, wherein a first tubular component and/or a second tubular component of the fixation sleeve being displaceable in longitudinal direction relative to each other from a first relative position to a second relative position such that the inner diameter of the internal mesh lumen is correspondingly reduced.

## Description

The invention relates generally to a fixation sleeve for an implantable medical lead and an assembly comprising the implantable medical lead and the fixation sleeve as well as an assembly comprising the fixation sleeve and a thread.

Medical devices such as cardiac pacemakers or defibrillators comprise at least one implantable medical lead emanating from the medical device and terminate at a predefined treatment location on the tissue. The lead applies electrical pulses to the predefined treatment location and/or transmits electrical signals detected at the predefined tissue location to the medical device. A fixation sleeve is used to secure the implanted lead near the treatment location at a pre-defined fixation location. Such fixation sleeve is generally configured as tubular member, the cavity or lumen of which is adapted to sheathe the electrically conductive lead body of the implantable medical device. A fixation sleeve usually also includes circumferential grooves adapted to receive a thread, e.g. a suture. The grooves facilitate wrapping the fixation sleeve with a thread to secure the sleeve to the body of a lead and to a patient's body tissue. Fixation sleeves are typically formed of soft, implantable elastomer material such as silicone.

During implantation the lead body is sheathed within the fixation sleeve. Once the lead is properly positioned at the predefined treatment location at the patient's body, the fixation sleeve is slid down the lead body to a point where it is supposed to be fixed at the patient's body - the fixation location. Conventionally, at the fixation location the fixation sleeve is wrapped with a thread in the circumferential groove. The thread is pulled tight and tied by the health care practitioner (HCP, e.g. clinician, nurse, ...) to secure the fixation sleeve to the lead body so that the lead body does not move in longitudinal direction with regard to the fixation sleeve. Additionally, the fixation sleeve is secured by the thread to the body tissue at the fixation location. Securing the fixation sleeve in this manner provides permanent hemostasis and lead stabilization at the treatment location.

However, because a fixation sleeve is constructed of soft, pliable material, problems may occur during the above-described procedure or afterwards. On the one hand, if the HCP pulls the thread too tight when securing the sleeve to the lead body, the thread may cut through the soft material of the fixation sleeve thereby damaging the lead body. When this happens, the lead must be replaced. Unfortunately, damage to the lead is often not detected until after the implantation is complete. Accordingly, an additional surgery is required to fix the problem thereby ultimately increasing the total cost of the implantation procedure. On the other hand, there may be problems if the fixation sleeve is not securely fixed to the lead body and the lead body moves relative to the fixation sleeve within the patient's body. Additionally, as the thread is pulled by hand, the clamping force cannot be reproducible defined.

Document US 2005/0055062 A1 discloses a screwless system for connecting a probe to an active implantable medical device comprising an axial female housing able to receive a probe connector and a reversible mechanical retention system to secure the probe connector in the housing, for example, a rotary bolt equipped with a side cam surface. Since both, the probe and the retention system are located within the housing, the housing has large outer dimensions which is frequently not desired. The same applies to an apparatus for fixing an electrode known from EP 1 709 990 A2 having a sleeve, wherein the sleeve contains an elastic tube and two parts surrounding the tube, wherein the tube forms the inner longitudinal cavity for accommodating the electrode, wherein the two parts can be pulled apart in the axial direction counter to the restoring force of the elastic tube that holds them together, to an extent such that the two parts can be rotated by twisting or wringing the elastic tube and resecured in the new rotational position in contact with one another against rotary movements. The sleeve has the disadvantage that the force for fixing the electrode cannot precisely be controlled.

Accordingly, there is a need for a fixation sleeve that can be securely and reproducible fixed to the lead body even if the lead body slightly varies in diameter and that has small outer dimensions at the same time.

The above object is solved by a fixation sleeve with the features of claim 1 and by assemblies comprising a fixation sleeve and a lead or a thread with the features of claims 12 and 14.

In particular, the object is solved by a fixation sleeve for an implantable lead with a lead body, wherein the fixation sleeve has a substantially tubular shape comprising an internal lumen extending therethrough and extending in a longitudinal direction, wherein the fixation sleeve comprises a tubular mesh configured such that an internal mesh lumen forms a section of the internal lumen of the fixation sleeve, wherein the internal mesh lumen is configured to receive the lead body, wherein the fixation sleeve comprises a first tubular component, in particular a first rigid tubular component, and a second tubular component, in particular a second rigid tubular component, wherein the first tubular component is fixedly attached to a first longitudinal end of the tubular mesh and the second tubular component is fixedly attached to a second longitudinal end opposite the first longitudinal end of the tubular mesh via a spring component, wherein the first tubular component and/or the second tubular component being displaceable in longitudinal direction relative to each other from a first relative position to a second relative position thereby extending the mesh in longitudinal direction from a first length to a greater second length such that the inner diameter of the internal mesh lumen is correspondingly reduced, wherein in the first relative position a longitudinal movement of the lead body within the internal mesh lumen is allowed and, in contrast, in the second relative position a longitudinal movement of the lead body within the internal mesh lumen and thereby within the internal lumen of the fixation sleeve the is prevented.

The implantable fixation sleeve has a substantially tubular body that forms an internal lumen or bore extending therethrough for receipt and guidance of the body of the implantable medical lead. The internal lumen substantially extends in longitudinal direction and has an inner surface, wherein the lead and the fixation sleeve can perform a relative movement along the internal lumen in longitudinal direction when the first tubular component and the second tubular component are in their initial, first relative position. Accordingly, in this first relative position, the fixation sleeve can be moved along the lead body until it reaches the pre-defined fixation position.

As indicated above, the fixation sleeve comprises a tubular mesh forming an internal mesh lumen extending therethrough, wherein the internal mesh lumen substantially extends in longitudinal direction. The internal mesh lumen forms a section of the internal lumen of the fixation sleeve which means that the internal mesh lumen forms the internal lumen of the fixation sleeve along all or part of its length, wherein the length is the dimension of the internal lumen of the fixation sleeve in longitudinal direction. The tubular mesh comprises a first end along its longitudinal direction and a second end, wherein the second end is opposite the first end in longitudinal direction. In the following, the first end is referred to as first longitudinal end and the second end is referred to as second longitudinal end.

The fixation sleeve further comprises a first tubular component, in particular a first rigid tubular component, and a second tubular component, in particular a second rigid tubular component, wherein the first tubular component is fixedly attached to the first longitudinal end of the tubular mesh and the second tubular component is fixedly attached to the second longitudinal end of the tubular mesh via a spring component. Accordingly, a movement of the first tubular component and/or the second tubular component is transmitted to the respective first or second longitudinal end of the tubular mesh thereby extending or compressing the tubular mesh. The spring component is accommodated between the second longitudinal end of the tubular mesh and the second tubular component, in the way that the second longitudinal end of the tubular mesh is attached to a first end of the spring component and a second end of the spring component is attached to the second tubular component. The second end of the spring component may be opposite the first end of the spring component in longitudinal direction. The spring component functions as a force/displacement buffer. When the inner mesh diameter is reached at which the tubular mesh clamps the lead body, the force for stretching the mesh increases significantly. In this case, the spring provided in series with the tubular mesh stores additional elongation.

The fixation sleeve is further configured such that the first tubular component and/or the second tubular component being displaceable in longitudinal direction relative to each other from the first relative position to a second relative position thereby extending the mesh in longitudinal direction from a first length to a greater second length such that the inner diameter of the internal mesh lumen is correspondingly reduced. In the first, initial relative position the lead body is movable within the tubular mesh and therefore within the fixation sleeve in longitudinal direction. In the second relative position a longitudinal movement of the lead body is prevented. Due to extension of the mesh in longitudinal direction starting from the first relative position of the first and second tubular components, the shape of the internal mesh lumen is changed and its inner diameter is reduced to such an extent that the inner diameter of the internal mesh lumen corresponds to the outer diameter of the lead body and provides a clamping force to the lead body. In one embodiment of the fixation sleeve the tubular mesh is configured such that in the second relative position of the first tubular component and the second tubular component the tubular mesh tightly clamps the lead body and in the first relative position the inner surface of the internal mesh lumen has a distance from the lead body. As indicated above, in the second relative position, the mesh provides a pre-defined clamping force to the lead body thereby clamping the lead body and fixing the fixation sleeve relative to the lead body. The mesh mechanically transmits the clamping force to the lead body. The fixation sleeve not only exactly defines the clamping force by the internal mesh lumen's cross section but also the clamping path by the shape and the extension of the internal lumen in longitudinal direction. Due to the structure of the tubular mesh, the clamping force is provided uniformly along the full length of the mesh to the lead body since each individual mesh segment functions as clamping partner forming a frictional connection with the clamped lead body that is adapted to the outer shape and diameter of the lead body. Altogether a greater and more defined clamping force can be achieved, in particular a pre-defined fixation of the lead body with a predefined clamping force and/or predefined geometric deformation within the fixation sleeve can be provided that extends along the full length of the tubular mesh in longitudinal direction. Further, the fixation can be realized by a simple action of the HCP during implantation procedure, namely a relative longitudinal movement of the first component and/or of the second component. Additionally, the simple action only causes a change of the internal lumen and, where appropriate, with a small extension of the overall length of the fixation sleeve so that the fixation sleeve has minimal space requirement. The inventive solution may be very easily adapted to different lead body diameters. All process steps during manufacturing and implantation are simple to realize and are based on in principle known process steps and standard components.

In one embodiment, the tubular mesh is configured such that a minimal internal mesh diameter of the tubular mesh may be smaller than a smallest possible outer diameter of the lead body (used with the fixation sleeve) in a section where the fixation sleeve is usually fixed to the lead body and if manufacturing tolerances of the lead are taken into account. The extension of the tubular mesh can be provided up to a maximum length (in longitudinal direction) that corresponds to a minimal internal mesh diameter. A further extension of the tubular mesh to a length greater than the maximum length requires considerably increased forces. Accordingly, the pre-defined clamping force to the lead body can only be provided if the smallest possible outer diameter of said lead body section is greater than the minimal internal mesh diameter. A further tension force provided by the HCP at the tubular mesh is stored by the spring component. Accordingly, damaging of the tubular mesh or any other component of the fixation sleeve is prevented.

In one embodiment of the fixation sleeve the first tubular component and the second tubular component are configured such that their second relative position is securely fixed. Accordingly, the clamping force can be permanently provided by the tubular mesh. The fixation of the first tubular component and/or the second tubular component in their second relative position may be provided by an interlocking comprising a positive locking connection, e.g. a ratchet mechanism, and/or a frictional connection. In one embodiment of the fixation sleeve the fixation of the first tubular component relative the second tubular component in the second longitudinal position may be detachable or permanent. Additionally, tensile forces from the lead body are transmitted to the fixation sleeve without causing geometric changes at the fixation sleeve. Additional tensioning of the mesh would lead to deviations in the clamping forces. Accordingly, the above described adjustable length locking the mesh or the second relative position of the first and second tubular components is required to maintain the preload providing the clamping.

In one embodiment of the fixation sleeve the first tubular component and/or the second tubular component and/or the spring component is composed of at least two elements, wherein one of these elements may be fixed to another one of these elements and consist of identical, similar or different materials. Such composite design of the fixation sleeve allows to provide a protective cover of the tubular mesh and a simple manufacturing of the fixation of the second relative position of the first tubular component and the second tubular component for different lead body diameters.

In one embodiment of the fixation sleeve, the first tubular component comprises a first rigid ring-like or tubular element providing a fixation of the tubular mesh at its first longitudinal end and a first sleeve-like element extending from the first rigid ring-like or tubular element longitudinally into the direction of the second tubular component covering the tubular mesh. The spring component comprises a second rigid ring-like or tubular element provided for fixation of the tubular mesh at its second longitudinal end and a tension spring, e.g. a coil tension spring, attached to and extending in series from the second ring-like or tubular element. The second tubular component comprises a third rigid ring-like or tubular element attached to the second end of the tension spring and a second sleeve-like element extending from the third rigid ring-like or tubular element longitudinally into the direction of the first tubular component covering the tension spring. The first sleeve-like element and the second sleeve-like element are configured to provide a permanent fixation of the first tubular component and the second tubular component in the second relative position by a positive locking connection (e.g. a ratchet mechanism) and/or by a frictional connection. The first sleeve-like element may cover one section of the second sleeve-like element or the other way around. The inner one of the first sleeve-like element and the second sleeve-like element may be slitted in longitudinal direction, whereas the outer one has a full sleeve-like shape thereby realizing a complete covering of the inner components. In case the first sleeve-like element and the second sleeve-like element form a ratchet mechanism, one of the first and second sleeve-like elements comprises teeth projecting from its surface opposite the other sleeve-like element along its longitudinal length that may intermesh with at least one pawl extending from the opposite surface of the other sleeve-like element.

In one embodiment of the fixation sleeve, the first tubular component comprises a first rigid ring-like or tubular element providing a fixation of the tubular mesh at its first longitudinal end and an integral first sleeve-like element extending from the first rigid ring-like or tubular element longitudinally into the direction of the second tubular component covering one section of the tubular mesh. The spring component comprises a second rigid ring-like or tubular element provided for fixation of the tubular mesh at its second longitudinal end and a compression spring, e.g. a coil compression spring, attached to and extending in series from the second ring-like or tubular element. The compression spring is arranged/positioned such that it overlaps and surrounds at least a section of the tubular mesh on the outside. The second tubular component comprises a third rigid ring-like or tubular element attached to the second end of the compression spring and a second sleeve-like element extending from the third rigid ring-like or tubular element longitudinally into the direction of the first end of the compression spring and the second end of the tubular mesh covering the compression spring and at least a section of the tubular mesh. The first sleeve-like element and the second sleeve-like element are configured to provide a fixation of the first tubular component and the second tubular component in the second relative position by a positive locking connection (e.g. a ratchet mechanism) and/or by a frictional connection. The first sleeve-like element may cover one section of the second sleeve-like element on its outside or the other way around. The inner one of the first sleeve-like element and the second sleeve-like element may be slitted in longitudinal direction, whereas the outer one has a full sleeve-like shape thereby realizing a complete covering of the inner components. In case the first sleeve-like element and the second sleeve-like element form a ratchet mechanism, the one of the first and second sleeve-like elements comprises teeth projecting from its surface opposite the other sleeve-like element along its longitudinal length that may intermesh with at least one pawl extending from the opposite surface of the other sleeve-like element.

As indicated above, in one embodiment of the fixation sleeve, the spring component may be designed as a coil tension spring or a coil compression spring. As one can derive from above embodiments, the fixation sleeve using a tension spring has a smaller total diameter than the fixation sleeve with the compression spring. However, the fixation sleeve comprising the compression spring is shorter in longitudinal direction than the one using a tension spring. Accordingly, the design of the fixation sleeve may be adapted to the specific requirements of the patient or medical application.

As further indicated above, in one embodiment of the fixation sleeve, the spring component is arranged in series with the tubular mesh along the longitudinal direction and/or at least partially overlaps the tubular mesh (i.e. the spring component overlaps the tubular mesh longitudinally along a section). These are different construction principles which can be realized using the inventive fixation sleeve.

In one embodiment the internal lumen of the fixation sleeve may be formed by the internal mesh lumen and, if applicable, additionally by an internal lumen of at least one of the group of components comprising the first tubular component, the second tubular component, the spring component and elements of these components.

In one embodiment of the fixation sleeve the tubular mesh comprises or consists of a tubular wire braid consisting of at least two intersecting wires, e.g. eight intersecting wires or sixteen intersecting wires, and/or a tubular mesh having flexible intersections. Intersecting wires may be connected to each other in three different ways (braided/intertwined, coiled/wound or cross-linked/crossing pints connected). They are either intertwined, which means that they regularly overlap and underlap each other in the axial and radial directions. The second option for the wires is to be wound, which means that they lie on top of or next to each other but do not alternate the overlap. The third option, which is only used for wires with larger diameters, is that the crossing points are non-detachably connected to each other (welded as with a structural steel mesh). The wires then form a mesh/net and no longer exist as individual wires. In order to achieve the desired reduction in diameter, braided and wound wires must be able to move against each other. In a net structure, the diameter can only be reduced if the connections (wires) between the mesh/net nodes can bend at the intersections (flexible, bendable connection to the intersections). This principle is often used for packaging nets where the contents are very irregularly shaped (apple sine nets). The use of a net structure for the wrapping element is particularly suitable for plastic injection molded parts. For the present case, both braided, coiled and cross-linked arrangements of wires are possible.

In the latter case the first tubular component, the second tubular component, the tubular mesh and the spring component may be manufactured by injection molding, wherein, for example, at least two of them form one integral assembly. The wires of the wire braid may consist of or comprise a material of the group comprising stainless steel, nylon, carbon, Nickel Cobalt alloy, e.g. MP35N, Kevlar, PEEK, PET, Nitinol, Tungsten alloy, Platinum, Platinum alloy, and all filamentary materials (metals, plastics, etc.) that can be wound or braided. When using a mesh, injection molding is another method available for producing a wrapping element / wire braid. Such wire may have a diameter from 0.02 mm to 0.5 mm, in particular from 0.02 mm to 0.1 mm, e.g. from 0.03 mm to 0.08 mm. The wire of a wire braid may consist of a strand of a plurality of wires, e.g. a strand of parallel wires, analogue to a shielding braid of electrical supply lines. The wire of the wire braid may have a circular or polygonar (e.g. triangular, rectangular) cross-sectional shape. Each one of such tubular wire braid must decrease its diameter of the inner lumen if it is extended (i.e. increases its length) in longitudinal direction.

In one embodiment of the fixation sleeve the first tubular component and the second tubular component are pivotable relative to each other about the longitudinal axis to arrive at at least two different pre-defined second relative positions provided along the circumference of the first tubular component or the second tubular component. Accordingly, the pre-defined length of the tubular mesh in the second relative position of the first tubular component and the second tubular component can be varied to a greater extend or in smaller steps as these steeps are distributed over the circumference of one of the first and second tubular component.

In one embodiment of the fixation sleeve the first tubular component and/or the second tubular component comprises a screw-nut combination configured to adapt the length of the respective component to the displacement of the first tubular component and/or the second tubular component in longitudinal direction relative to each other. One element of the screw-nut-combination may be provided, for example at the first rigid ring-like or tubular element of the first tubular component. The other element of the screw-nut combination may be provided at the first sleeve-like element thereby providing an adaption of the overall length of the fixation sleeve in a desired way, e.g. after taking the second relative position and/or to the overall length in the first relative position of the first and second tubular components. The nut of the screw-nut combination can be screwed along the screw for length adaption.

The above problem is further solved by an assembly comprising a lead for a medical device (such as a pacemaker, defibrillator, neurostimulator) and a fixation sleeve as described above, wherein the fixation sleeve sheathes a body section of the lead and is movable along the lead body in longitudinal direction as long as the first component and the second component are in the first relative position. The assembly with the fixation sleeve and the lead has the above described advantages and embodiments. Prior implantation and prior fixation of the fixation sleeve to the lead body and the patient's tissue, the fixation sleeve can be moved along the lead body in the longitudinal direction to adapt the position of the fixation sleeve to the personal needs of the patient. During implantation procedure, first, the tip (electrode) of the lead is properly placed at the predefined treatment position. Then, if necessary, after sliding the fixation sleeve along the lead body, the fixation sleeve is attached to the fixation location. In one embodiment of the assembly, the lead comprises an electrically conducting tip at its distal end and a connector for electrical and mechanical connection to the medical device at its proximal end.

The above problem is further solved by an assembly comprising a thread (suture) and a fixation sleeve as described above, wherein the thread and the fixation sleeve are configured such that during implantation procedure the thread is used to fix the fixation sleeve at a pre-defined target location within the patient's body. The assembly with the fixation sleeve and the thread has the above described advantages and embodiments (with regard to the fixation sleeve). The suture may comprise or consist of at least one material of the group comprising Polypropylen (PP), Polytetrafluorethylen (PTFE) or a polyester, e.g. polyethylene terephthalate (PET). The diameter of the thread may be, for example, 1 Ph. Eur. to 8 Ph. Eur (1 Ph. Eur. = 0,1mm). The fixation sleeve may comprise a groove at the outer surface of the tubular body to guide the thread during/after implantation and to prevent slipping of the fixation sleeve relative to the patient's body.

The above fixation sleeve and assemblies can be cost-effectively produced because standard components can be used. The fixation sleeve can achieve a high clamping force, is tolerant with regard to deviations in the lead body diameter and/or lead body shape without clamping force reduction. The tubular mesh of the fixation sleeve provides a great friction surface leading to improved distribution of forces on the surface of the lead body. Penetration of the tubular mesh into the surface of the lead body may be limited by the preload of the spring component.

The present invention will now be described in further detail with reference to the accompanying schematic drawings, wherein
- Fig. 1: shows a patient with an example medical device, lead and fixation sleeve in a front view,
- Fig. 2: depicts a longitudinal section of a first embodiment of a fixation sleeve in a first relative position with an implantable lead extending through the fixation sleeve,
- Fig. 3: illustrates the assembly and view of Fig. 2 in a second relative position,
- Fig. 4: shows a first relative position of components of a second embodiment of a fixation sleeve with an implantable lead extending through the fixation sleeve in a side view,
- Fig. 5: depicts the first relative position of the embodiment of Fig. 4 having a screw-nut combination in a side view,
- Fig. 6: illustrates the first relative position of the embodiment of Fig. 4 fully assembled in a side view,
- Fig. 7: shows the second relative position of the embodiment of Fig. 4 fully assembled in a perspective side view,
- Fig. 8: illustrates a tensile test using a prototype of the embodiment of Fig. 4 to 7, wherein the x-axis of the diagram shows the strain and the y-axis shows the stress.
- Fig. 9: shows a longitudinal section of a third embodiment of a fixation sleeve in a first relative position with an implantable lead (depicted by dashed lines) extending through the fixation sleeve,
- Fig. 10: illustrates the assembly and view of Fig. 9 in a second relative position,
- Fig. 11: depicts a tubular mesh and an implantable lead extending therethrough used for determination of characteristic values,
- Fig. 12: shows a diagram illustrating the tension path of tubular meshes of different initial lengths over the inner mesh diameter of the respective tubular mesh,
- Fig. 13: shows a diagram illustrating the relative strain of the tubular meshes of different initial lengths of Fig. 12 over the clamping diameter.

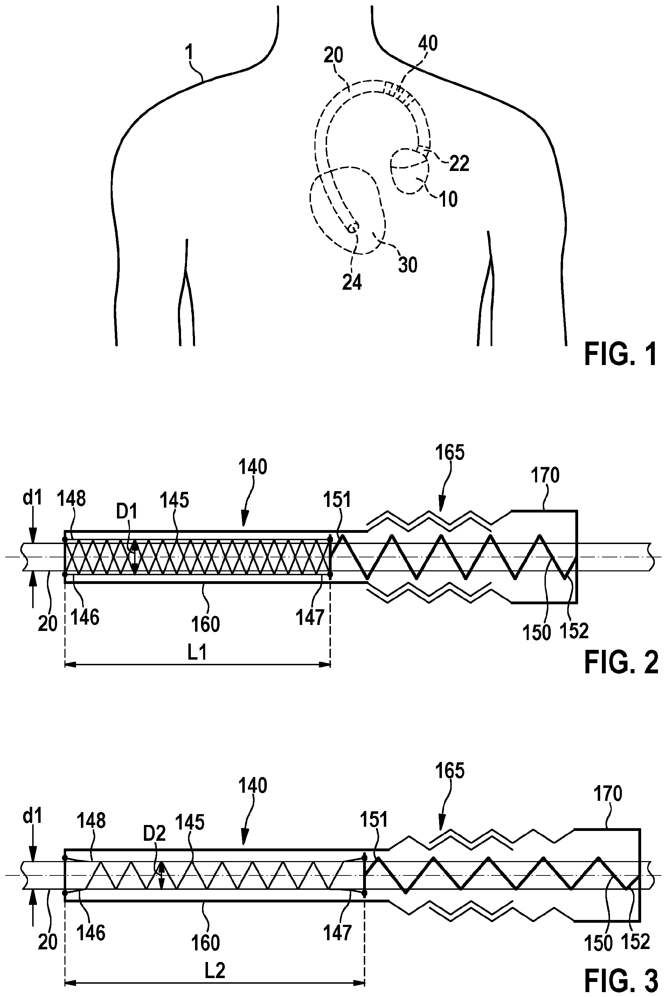

Referring to the drawings, Fig. 1 shows a pacemaker system including a pacemaker 10 of a patient 1, and an electrode lead 20 electrically and mechanically connected to the pacemaker 10 by a connector 22. A distal tip of the lead 20 with an electrode 24 is positioned within a heart 30 of the patient 1 at a predefined treatment position. The lead 20 passes through a fixation sleeve 40 which in turn is sutured into surrounding tissue of the patient 1 at a predefined fixation position using a thread (e.g. a suture) to help position and stabilize the location of lead 20 and electrode 24.

A first embodiment of a fixation sleeve 140 is shown in Fig. 2 and 3, wherein a lead 20 extends through an inner lumen of the fixation sleeve 140 extending in a longitudinal direction. The longitudinal direction and axis of the inner lumen of the fixation sleeve 140 is depicted by the dot-dashed line. The fixation sleeve 140 comprises a tubular mesh 145, a spring component 150 (i.e. a coil tension spring), a first tubular component 160 and a second tubular component 170. A first longitudinal end 146 of the tubular mesh 145 is fixed to the first tubular component 160 and a second longitudinal end 147 of the tubular mesh 145 is fixed to the first end 151 of the spring component 150. The second end 152 of the spring component 150 is fixed to the second tubular component 170. The first tubular component 160 covers the tubular mesh 145 and extends longitudinally into the direction of the second tubular component 170. The second tubular component 170 covers the spring component 150 at least partly and extends longitudinally into the direction of the first tubular component 160. In a section in which the second tubular component 170 overlaps the first tubular component 160 the first tubular component 160 and the second tubular component 170 form a positive locking connection 165, e.g. a ratchet, whose interlocking sections are indicated schematically by parallel zig-zag lines.

Fig. 2 depicts the fixation sleeve 140 in its initial, first relative position in which the body of lead 20 can be moved within the inner lumen of the fixation sleeve. The inner diameter D1 (e.g. 3 mm) of the inner mesh lumen 148 of the tubular mesh 145 is slightly greater than the outer diameter d1 (e.g. 2 mm) of the body of lead 20. The tubular mesh may be a braid of 16 stainless steel wires, each having a diameter of, e.g. 0.05 mm. The initial length of the tubular mesh 145 is L1 (e.g. 10 mm. For fixation of the fixation sleeve 140 at the lead 20 the HCP moves the second tubular component 170 longitudinally away from the first tubular component 160 (i.e. relative to the first tubular component 160) into a second relative position such that the length of the tubular mesh 145 increases to a value L2 (L2 > L1) (L2, e.g. 18 mm and, accordingly, the inner diameter of inner mesh lumen 148 of the tubular mesh 145 decreases to a value D2 (D1 > D2) (D2 e.g. 2 mm) and provides a clamping force to the body of lead 20 as one can derive from Fig. 3. The value D2 of the inner diameter of the inner mesh lumen 148 corresponds the outer diameter d1 of the body of lead 20. Fig. 3 illustrates the fixation sleeve 140, wherein the first and second tubular components 160, 170 take their second relative position. This position is illustrated by a different position of the zig-zag lines of the positive locking connection 165. The parallel zig-zag lines do also show that the first and second tubular components 160, 170 are locked in this second relative position. The spring component 150 serves as a reservoir for maintaining the pre-tension.

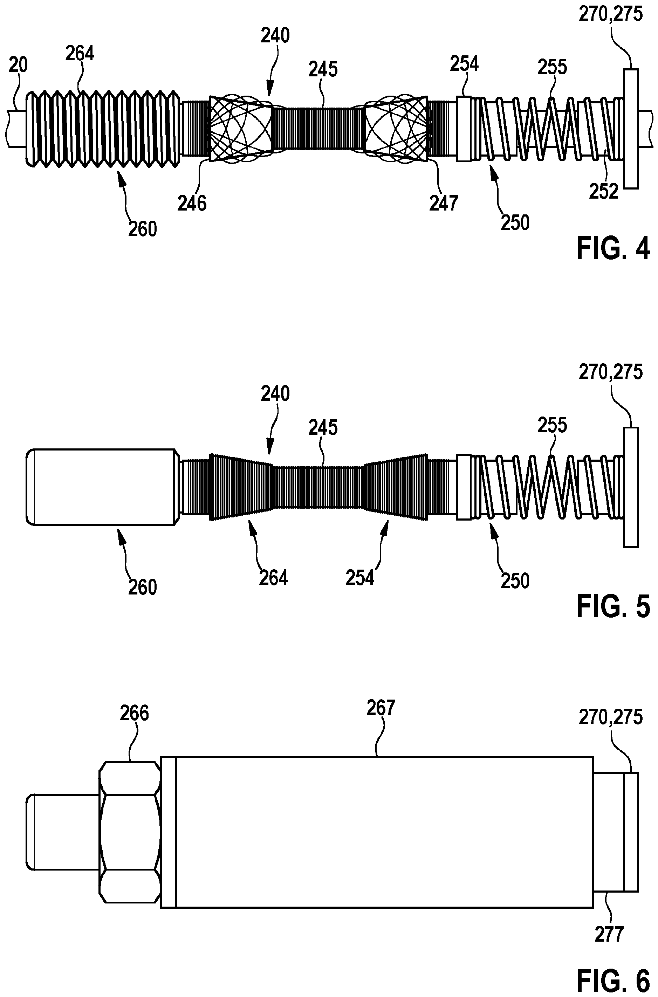

The second embodiment of a fixation sleeve 240 shown in Fig. 4 to 7 basically corresponds to the first embodiment depicted in Fig. 2 to 3. The difference between both embodiments is that in the embodiment of Fig. 4 to 7 each component consists of several elements.

The fixation sleeve 240 comprises a tubular mesh 245 having a first end 246, a second end 247 and an inner mesh lumen forming a section of the inner lumen of the fixation sleeve.

The spring component 250 of the fixation sleeve 240 having a coil tension spring 255 further comprises a first rigid tubular element 254 forming a rigid connection between the second end 247 of the tubular mesh 245 and the coil tension spring 255. The first tubular element 254 has a first section having an outer shape of a truncated cone and three further sections, each having an outer shape of a cylinder with different diameter. As indicated in Fig. 4, the truncated cone section and the adjacent step is utilized for fixation of the tubular mesh 245. The opposite cylindrical section (in longitudinal direction) is configured for attachment of the coil tension spring 255.

The first tubular component 260 comprises a second tubular element 264, a nut 266 and a first cylindrical sleeve 267. The second tubular element 264 comprising a truncated cone section is configured for fixation of the first end 246 of the tubular mesh 245. The second tubular element 264 further comprises a threaded portion, wherein the thread is provided at the outer surface of the second tubular element 264.. The nut 266 can be screwed relative to the second tubular element 264 using the threaded connection between the second tubular element 264. The HCP may grip the combination of elements 266, 267 using the nut 266 and screw them relative the second tubular element 264. Thereby, the overall length of the fixation sleeve 240 can be adapted, e.g. after transformation from the first to the second relative position of the first and second tubular components 260, 270. Additionally, the first cylindrical sleeve 267 cover the internal elements of the fixation sleeve 240, wherein the first cylindrical sleeve 267 covers the tubular mesh 245.

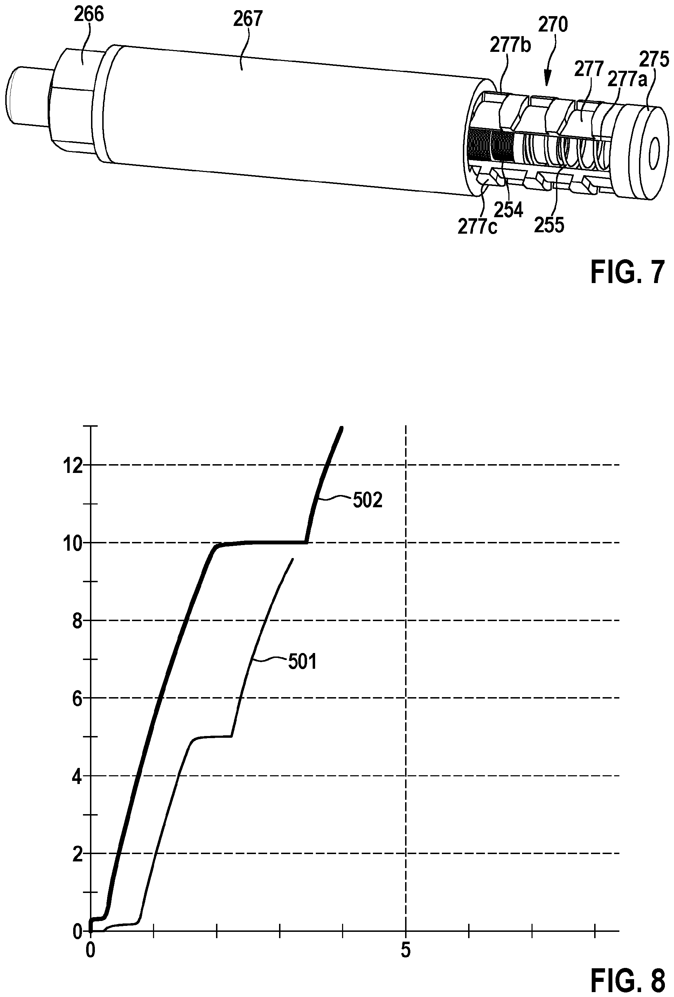

The second tubular component 270 comprises a second cylindrical element 275 and a second cylindrical sleeve 277 fixed to the cylindrical element 275. The second cylindrical element 275 has a stepped shape that looks like a "T" when viewed from the side (see Fig. 5). It is configured to fix the second end of the coil tension spring 255. The second cylindrical sleeve 277 is best illustrated in Fig. 7 and comprises two longitudinal slits 277a. The second cylindrical sleeve 277 covers the coil tension spring 255 and comprises sloping teeth 277b and 277c projecting from its outer surface. The slits 277a and the teeth 277b and 277c intermesh with at least one corresponding pawl extending from the opposite, inner surface of the first cylindrical sleeve 267. The at least one pawl, e.g. two pawls, may be moved in longitudinal direction within the slits 277a so that the second tubular component 270 can be displaced relative the first tubular component 260 to extend the tubular mesh 245. With sufficient elongation of the tubular mesh 245 to clamp the body of lead 20, the second tubular member 270 may be pivoted such that the at least one pawl engages one of the teeth 277b or 277c, wherein the teeth 277b and the teeth 277c are located offset along the circumference of the second tubular member 270, i.e. its second cylindrical sleeve 277 so that different second relative positions of the fixation sleeve 240 can be realized. Fig. 6 shows the initial first relative position of the first tubular component 260 and the second tubular component 270 in which the fixation sleeve 240 can be moved along the body of lead 20, whereas Fig. 7 depicts the second relative position in which the fixation sleeve 240 is fixedly clamped to the body of the lead 20 by the reduction of the inner diameter of the inner mesh lumen of the tubular mesh 245.

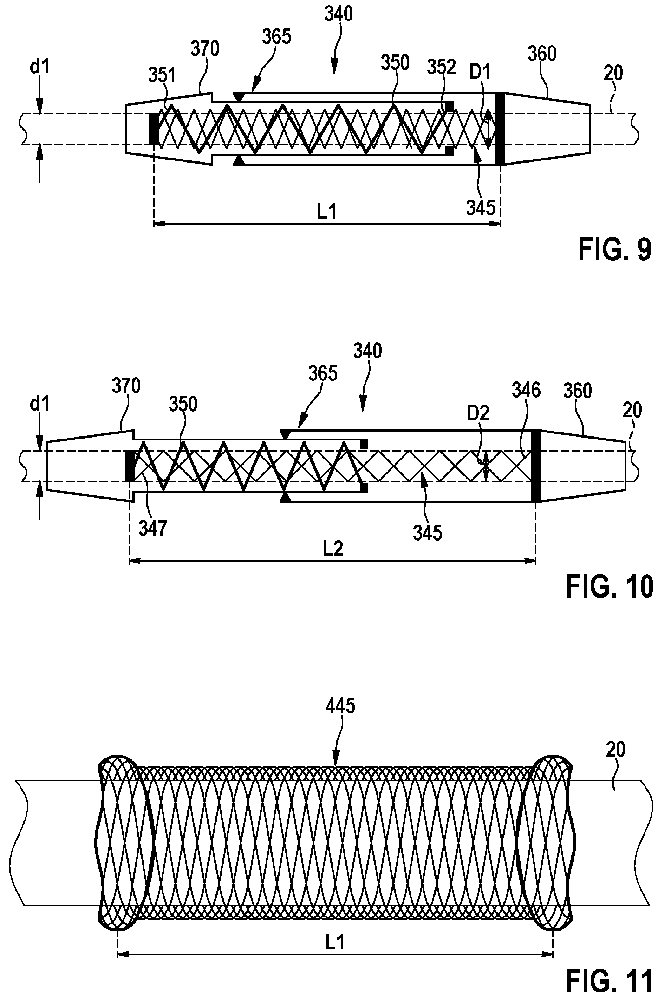

The third embodiment of a fixation sleeve 340 shown in Fig. 9 and 10 is basically similar to the first embodiment of Fig. 2 and 3. Accordingly, the elements and components of the fixation sleeve 340 correspond to the elements and components of the fixation sleeve 140. It is therefore referred to above explanation of the first embodiment (fixation sleeve 140) with regard to Fig. 2 and 3. The differences are indicated below. The elements and components of fixation sleeve 140 that correspond to elements and components of fixation sleeve 340 have reference numbers that differ by 200. For example, the tubular mesh 345 of fixation sleeve 340 corresponds to the tubular mesh 145 of fixation sleeve 140. The dimensions D1, D2, L1, L2 and d1 defined with regard to fixation sleeve 140 are indicated with regard to fixation sleeve 340 with identical reference numbers.

One difference of fixation sleeves 140 and 340 is that the first end 346 of the tubular mesh 345 is located at the right side of Fig. 9 and 10 and the second end 347 of the tubular mesh 345 at the left side. Furthermore, the spring component 350 is a compression spring that is fixed to and extends with its first end 351 from the second end 347 of the tubular mesh 345 in longitudinal direction such that it runs parallel to and overlaps the tubular mesh 345. The second tubular component 370 is fixed to the opposite end (i.e. the second end 352) of the spring component 350 such that it covers the spring component 350 and partially the tubular mesh 345. The initial state in which the first tubular component 360 and the second tubular component 370 take the first relative position, wherein the tubular mesh 345 has a first length L1 and a first inner diameter D1 of the inner mesh lumen is depicted in Fig. 9. Fig. 10 shows the second relative position of these components. In the first relative position, the length L1 of the tubular mesh is shorter than the length L2 in the second relative position. Accordingly, in the first relative position, the inner diameter D1 of the inner mesh lumen is greater than the inner diameter D2 in the second relative position.

Each of above embodiments of a fixation sleeve 140, 240, 340 may comprise at least one groove or eyelet for guiding a thread to fix (e.g. suture) the fixation sleeve to the patient's body at a pre-defined fixation position.

The clamping force of a prototype of a fixation sleeve as depicted in Fig. 4 to 7 was used in a tensile test in which a tensile force of the internal specification was provided for 60 seconds between the body of lead 20 clamped by the fixation sleeve 240 in the second relative position. Thereafter, the tensile force was increased and then the test was manually finished. For the test, the fixation sleeve was used horizontally in a tensioned state. Inside was a lead-like structure. A time and force-controlled testing machine was used. The test was carried out at room temperature and the friction partners were dry.

The measured strain is shown in Fig. 8, curve 501. In the second test a tensile force of the double of the internal specification was provided for 60 seconds and then the tensile force was increased and then the test was manually finished. The measured strain is shown in Fig. 8, curve 502. No relative movement of the tubular mesh 245 of the prototype and the body of lead 20 was observed in either test, the measured elongation results solely from the elongation of the lead 20 under load. Additionally, only shallow relief in the surface of the body of the lead 20 created by the tubular mesh 245 was observed, and the relief receded afterwards. The following applies to the diagram in Fig.8 and the following diagrams: The curves are typical and not dependent on specific dimensions.

To visualize the behavior of the tubular mesh at different positions of the first and second tubular components and to receive characteristic values regarding length and inner diameter change as well as strain is measured using examples of a tubular mesh 445 as depicted in Fig. 11 in different initial lengths, e.g. 3 mm, 6 mm or 10 mm. The tubular mesh 445 is located at a body of a lead 20 and may have different diameter, e.g. 1.5 mm or 2 mm.

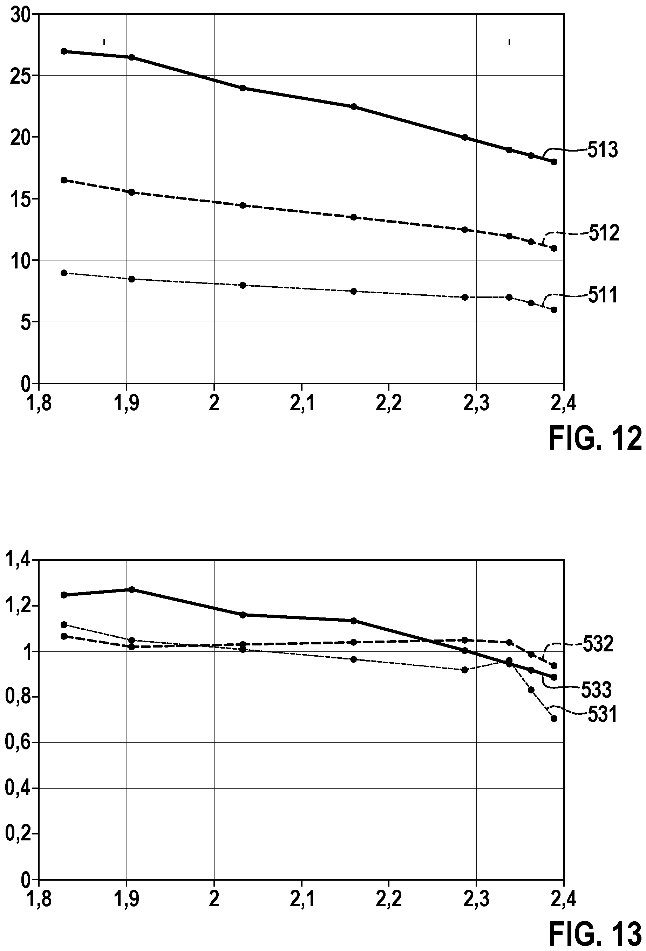

Two diagrams containing measurement values for these tubular meshes 445 and leads 20 are shown in Fig. 12 and 13. As already mentioned, the curves are typical and not dependent on specific dimensions.

The diagram of Fig. 12 illustrates the tension path of tubular meshes having the initial length L1 (curve 511), of L2 approximately 2*L1 (curve 512) and of L3 approximately 3*L1 (curve 513) and an initial inner mesh diameter of > 2 mm over the inner mesh diameter of the respective tubular mesh. It can be derived, for example, from curve 513 that he inner mesh diameter reduction from 2 mm to 1.8 mm for the tubular mesh having an initial length of 10 mm is provided by an elongation of the tubular mesh by, for example, approximately 8 mm.

From Fig. 13 one can derive the relative strain (elongation of the fixation sleeve of calculated mounting length) of the tubular meshes of different initial lengths of L1 (curve 531), of L2 (curve 532) and of L3 (curve 533) over the clamping diameter. The shape of the curves is a characteristic for the geometric and mechanical mesh design (i.e. braid design).

## Claims

1. A fixation sleeve (40, 140, 240, 340) for an implantable lead (20) with a lead body, wherein the fixation sleeve (40, 140, 240, 340) has a substantially tubular shape comprising an internal lumen extending therethrough and extending in a longitudinal direction, wherein the fixation sleeve (40, 140, 240, 340) comprises a tubular mesh (145, 245, 345) configured such that an internal mesh lumen (148) forms a section of the internal lumen of the fixation sleeve (40, 140, 240, 340), wherein the internal mesh lumen (148) is configured to receive the lead body, wherein the fixation sleeve (40, 140, 240, 340) comprises a first tubular component (160, 260, 360) and a second tubular component (170, 270, 370), wherein the first tubular component (160, 260, 360) is fixedly attached to a first longitudinal end (146, 246, 346) of the tubular mesh (145, 245, 345) and the second tubular component (170, 270, 370) is fixedly attached to a second longitudinal end (147, 247, 347) opposite the first longitudinal end (146, 246, 346) of the tubular mesh (145, 245, 345) via a spring component (150, 250, 350), wherein the first tubular component (160, 260, 360) and/or the second tubular component (170, 270, 370) being displaceable in longitudinal direction relative to each other from a first relative position to a second relative position thereby extending the tubular mesh (145, 245, 345) in longitudinal direction from a first length (L1) to a greater second length (L2) such that the inner diameter (D 1, D2) of the internal mesh lumen (148) is correspondingly reduced, wherein in the first relative position a longitudinal movement of the lead body within the internal mesh lumen (148) is allowed and in the second relative position a longitudinal movement of the lead body is prevented.

2. The fixation sleeve (40, 140, 240, 340) of claim 1, wherein the first tubular component (160, 260, 360) and the second tubular component (170, 270, 370) are configured such that their second relative position is securely fixed.

3. The fixation sleeve (40, 140, 240, 340) of claim 2, wherein the secure fixation of the first tubular component (160, 260, 360) and/or the second tubular component (170, 270, 370) may be detachable or permanent.

4. The fixation sleeve (40, 140, 240, 340) of any one of the claims 2 to 3, wherein the fixation in the second relative position is provided by a positive locking connection, e.g. a ratchet mechanism (277b, 277c).

5. The fixation sleeve (40, 140, 240, 340) of any one of the previous claims, wherein the tubular mesh (145, 245, 345) is configured such that in the second relative position of the first tubular component (160, 260, 360) and the second tubular component (170, 270, 370) the tubular mesh (145, 245, 345) tightly clamps the lead body and in the first relative position the inner surface of the internal mesh lumen (148) has a distance from the lead body.

6. The fixation sleeve (40, 140, 240, 340) of any one of the previous claims, wherein the first tubular component (160, 260, 360) and/or the second tubular component (170, 270, 370) is composed of at least two elements (254, 255, 264, 266, 267, 275, 277).

7. The fixation sleeve (40, 140, 240, 340) of any one of the previous claims, wherein the spring component (150, 250, 350) is arranged in series with the tubular mesh (145, 245) along the longitudinal direction and/or at least partially overlaps the tubular mesh (345) along the longitudinal direction.

8. The fixation sleeve (40, 140, 240, 340) of any one of the previous claims, wherein the spring component (150, 250, 350) is or comprises a coil tension spring or a coil compression spring.

9. The fixation sleeve (40, 140, 240, 340) of any one of the previous claims, wherein the tubular mesh (145, 245, 345) comprises a tubular wire braid consisting of at least two wires and/or a tubular mesh having flexible intersections.

10. The fixation sleeve (40, 140, 240, 340) of any one of the previous claims, wherein the first tubular component (160, 260, 360), the second tubular component (170, 270, 370), the tubular mesh (145, 245, 345) and the spring component are manufactured by injection molding, wherein, for example, at least two of them form one integral assembly.

11. The fixation sleeve (340) of any one of the previous claims, wherein the first tubular component (360) and the second tubular component (370) are pivotable relative to each other about the longitudinal axis to arrive at at least two different pre-defined second relative positions provided along the circumference of the first tubular component (360) or the second tubular component.

12. The fixation sleeve (240) of any one of the previous claims, wherein the first tubular component (260) and/or the second tubular component (270) comprises a screw-nut combination configured to adapt the length of the respective component to the displacement of the first tubular component (260) and/or the second tubular component (270) in longitudinal direction relative to each other.

13. An assembly comprising a lead (20) for a medical device and a fixation sleeve (40, 140, 240, 340) according to any one of the claims 1 to 12, wherein the fixation sleeve (40, 140, 240, 340) sheathes a body section of the lead (20) and is movable along the lead body in longitudinal direction as long as the first tubular component (160, 260, 360) and the second tubular component (170, 270, 370) are in the first relative position.

14. The assembly of claim 13, wherein the lead (20) comprises an electrically conducting electrode member (24) at its distal end and a connector (22) for electrical and mechanical connection to the medical device (10), e.g. a pacemaker, at its proximal end.

15. An assembly comprising a thread and the fixation sleeve (40, 140, 240, 340) according to any one of the claims 1 to 12, wherein the thread and the fixation sleeve (40, 140, 240, 340) are configured such that during implantation procedure the thread is used to fix the fixation sleeve (40, 140, 240, 340) at a pre-defined target location within the patient's body.
